(19) Europäisches Patentamt
European Patent Office
Office européen des brevets

(11) Veröffentlichungsnummer: **0 011 768**
**B1**

(12) # EUROPÄISCHE PATENTSCHRIFT

(45) Veröffentlichungstag der Patentschrift: **21.10.81**

(51) Int. Cl.[3]: **C 07 D249/08**, A 61 K 31/41// C07C33/46

(21) Anmeldenummer: **79104483.7**

(22) Anmeldetag: **14.11.79**

(54) **Hydroxypropyl-triazole, Verfahren zu ihrer Herstellung und diese Verbindungen enthaltende Arzneimittel.**

(30) Priorität: **25.11.78 DE 2851086**

(43) Veröffentlichungstag der Anmeldung: **11.06.80 (Patentblatt 80/12)**

(45) Bekanntmachung des Hinweises auf die Patenterteilung: **21.10.81 Patentblatt 81/42**

(84) Benannte Vertragsstaaten: **AT BE CH DE FR GB IT LU NL SE**

(56) Entgegenhaltungen: **DE - A - 2 623 129**

(73) Patentinhaber: **BAYER AG**
**Zentralbereich Patente, Marken und Lizenzen**
**D-5090 Leverkusen 1, Bayerwerk (DE)**

(72) Erfinder: **Regel, Erik, Ing. grad**
**Bergerheide 72 a**
**D-5600 Wuppertal 1 (DE)**
Erfinder: **Büchel, Karl Heinz, Prof. Dr.**
**Bergerheide 62**
**D-5600 Wuppertal 1 (DE)**
Erfinder: **Haller, Ingo, Dr.**
**Viktoriastrasse 99**
**D-5600 Wuppertal 1 (DE)**
Erfinder: **Plempel, Manfred, Dr.**
**Palkestrasse 5**
**D-5600 Wuppertal 1 (DE)**

**EP 0 011 768 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Hydroxypropyl-triazole, Verfahren zu ihrer Herstellung und diese Verbindungen enthaltende Arzneimittel.

Die vorliegende Erfindung betrifft neue Hydroxypropyltriazole, mehrere Verfahren zu ihrer Herstellung sowie ihre Verwendung als Arznemittel, insbesondere als Antimykotika.

Es ist bereits bekannt geworden, daß Hydroxy-triazolyl-alkane, wie z.B. im Phenylteil substituierte 4,4-Dimethyl-3-hydroxy-2-phenoxy-1-(1,2,4-triazol-1-yl)-pentan-Derivate, gute antimykotische Wirkung aufweisen (vergleiche DE-A-23 50 121). Deren Wirkung ist jedoch, insbesondere gegen Dermatophyten, nicht immer ganz befriedigend.

Es wurden die neuen Hydroxypropyltriazole der allgemeinen Formel

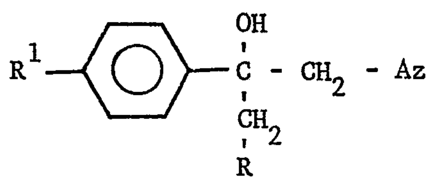

(I)

in welcher

Az für 1,2,4-Triazol-1-yl oder 1,3,4-Triazol-1-yl steht, und

R und $R^1$ für gegebenenfalls ein- oder zweifach durch Chlor substituiertes Phenyl stehen,

und deren physiologisch verträglichen Säureadditions-Salze gefunden. Sie weisen starke antimykotische Eigenschaften auf.

Weiterhin wurde gefunden, daß man die Hydroxypropyltriazole der Formel (I) erhält, wenn man

a) Triazolylmethyl-phenyl-ketone der Formel

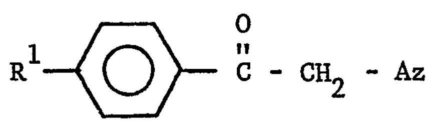

(II)

in welcher

Az und $R^1$ die oben angegebene Bedeutung haben,

mit einer Grignard-Verbindung der Formel

$$R - CH_2 - Mg - X \qquad (III)$$

in welcher

R die oben angegebene Bedeutung hat, und

X für Halogen, insbesondere Chlor oder Brom steht,

in Gegenwart eines Verdünnungsmittels umsetzt, oder

b) 1-Halogen-propan-2-ole der Formel

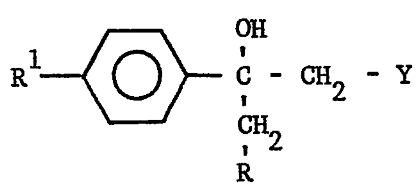

(IV)

in welcher

R und $R^1$ die oben angegebene Bedeutung haben, und

Y für Halogen, insbesondere Chlor oder Brom stehen,

mit Triazol, gegebenenfalls in Gegenwart eines Säurebindemittels und gegebenenfalls in Gegenwart eines Verdünnungsmittels, umsetzt. In manchen Fällen erweist es sich als vorteilhaft, das Triazol in Form eines Alkalisalzes, wie das Natrium- oder Kaliumsalz, einzusetzen.

Weiterhin können die erfindungsgemäß erhältlichen Hydroxypropyl-triazole der Formel (I) durch Umsetzen mit Säuren in die Salze überführt werden.

Überraschenderweise zeigen die erfindungsgemäßen Hydroxy-propyl-triazole eine bessere, therapeutisch nutzbare Wirksamkeit, insbesondere gegen Dermatophyten, als die aus dem Stand der Technik bekannten Hydroxy-triazolyl-alkane, wie z. B. im Phenylteil substituierte 4,4-Dimethyl-3-hydroxy-2-phenoxy-1-(1,2,4-triazol-1-yl)-pentan-Derivate, welche naheliegende Verbindungen sind. Die erfindungsgemäßen Wirkstoffe stellen somit eine wertvolle Bereicherung der Pharmazie dar.

Auch gegenüber der aus DE-A 26 23 129 bekannten Verbindung der Formel

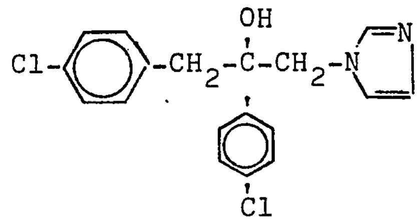

zeigen die erfindungsgemäßen Verbindungen bei sonst vergleichbar guter Wirkung gegen Trichophyton mentagrophytes in vitro und in vivo eine deutliche Überlegenheit.

Im Einzelnen seien außer den bei den Herstellungsbeispielen genannten Verbindungen die folgenden Verbindungen der Allgemeinen Formel (1) genannt (wobei Az jeweils für 1,2,4-bzw. 1,3,4-Triazol-1-yl steht) :

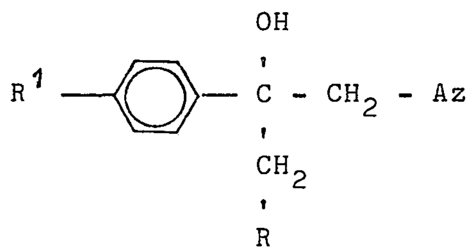

| R | $R^1$ |
|---|---|
| 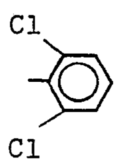 | 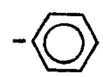 |
| 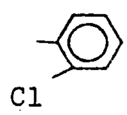 | 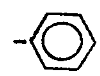 |
| 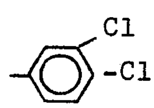 | 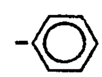 |
| 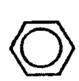 | 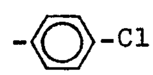 |
| 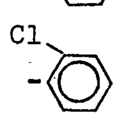 | 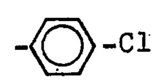 |
| 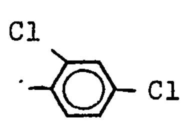 | 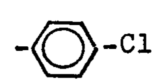 |
| 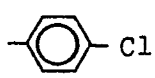 | 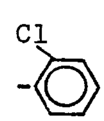 |

| R | $R^1$ |
|---|---|
| 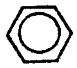 | 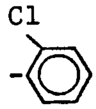 |
| 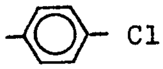 | 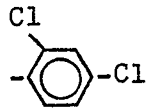 |
| 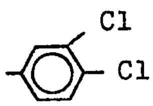 | 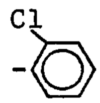 |
| 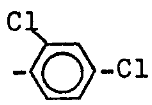 | 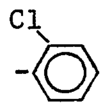 |
| 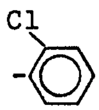 | 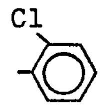 |
| 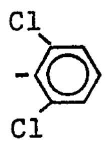 | 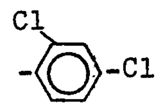 |
| 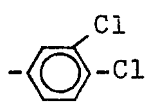 | 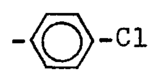 |
| 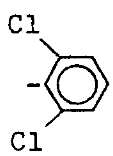 | 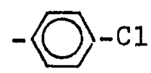 |

Verwendet man beispielsweise 4-Biphenylyl-(1,2,4-triazol-1-yl-methyl)-keton und 4-Chlorbenzylmagnesiumchlorid als Ausgangsstoffe, so kann der Reaktionsablauf durch das folgende Formelschema wiedergegeben werden (Verfahren a) :

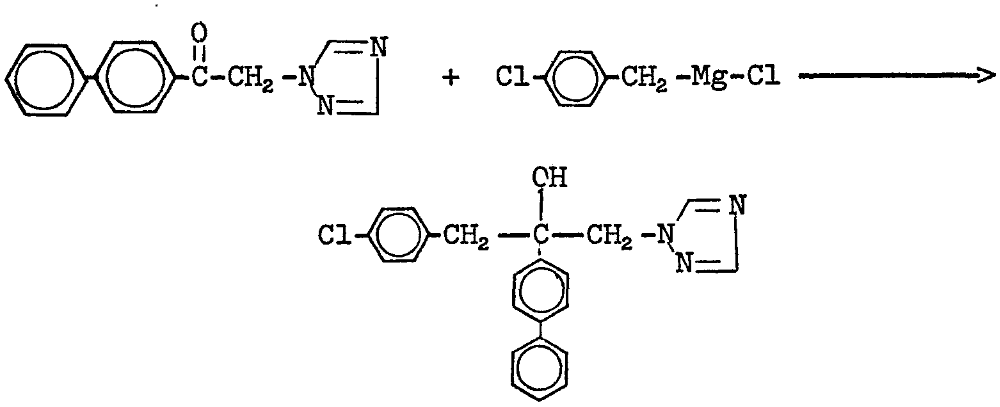

Verwendet man beispielsweise 2-(4-Biphenylyl)-3-chlor-1-(2,4-dichlorphenyl)-propan-2-ol und 1,2,4-Triazol-natrium als Ausgangsstoffe, so kann der Reaktionsablauf durch das folgende Formelschema wiedergegeben werden (Verfahren b) :

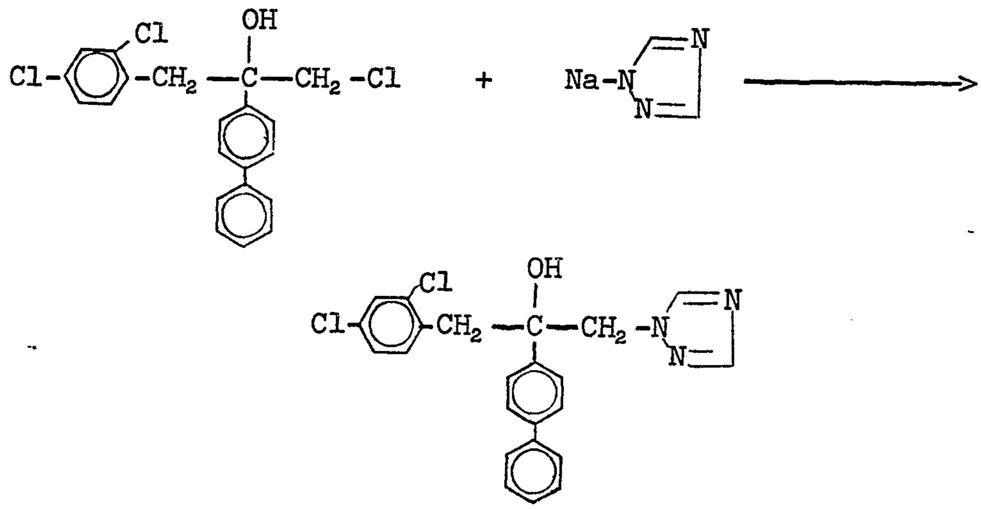

Die für die Verfahrensvariante (a) als Ausgangsstoffe zu verwendenden Triazolylmethylphenylketone sind durch die Formel (II) allgemein definiert.

Die Triazolylmethylphenylketone der Formel (II) sind noch nicht bekannt. Sie lassen sich jedoch in allgemein üblicher und bekannter Weise herstellen, indem man entsprechende Phenacyl-halogenide der Formel

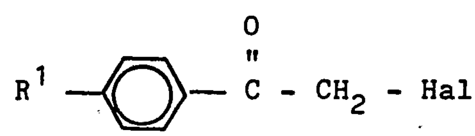 (VI)

in welcher

$R^1$ die oben angegebene Bedeutung hat und

Hal für Chlor oder Brom steht,

mit Triazol in Gegenwart eines Verdünnungsmittels, wie beispielsweise Dimethylformamid, und in Gegenwart eines säurebindenden Mittels, wie insbesondere einem Überschuß an Azol, bei Temperaturen zwischen 20 und 80 °C umsetzt (vergleiche hierzu auch die Angaben in der US-Patentschrift 3 658 813).

Die außerdem für die Verfahrensvariante (a) als Ausgangsstoffe zu verwendenden Grignard-Verbindungen sind durch die Formel (III) allgemein definiert. In dieser Formel steht *R* für die Reste, die bei den Verbindungen der Formel (I) bereits genannt wurden.

Die Grignard-Verbindungen der Formel (III) sind allgemein bekannte Verbindungen der organischen Chemie.

Die für die Verfahrensvariante (b) als Ausgangsstoffe zu verwendenden 1-Halogenpropan-2-ole sind durch die Formel (IV) allgemein definiert. In dieser Formel stehen *R* und $R^1$ für die Reste, die bei den Verbindungen der Formel (I) bereits genannt wurden.

Die 1-Halogenpropan-2-ole der Formel (IV) sind noch nicht bekannt. Sie lassen sich jedoch in allgemein üblicher und bekannter Weise herstellen, indem man Ketone der Formel (VI) mit Grignard-Verbindungen der Formel (III) entsprechend der Verfahrensvariante (a) umsetzt (vergleiche hierzu auch die Angaben in der DE-A-26 23 129 sowie die Herstellungsbeispiele).

Für die erfindungsgemäße Umsetzung nach Verfahren (a) kommen als Verdünnungsmittel alle für eine Grignard-Reaktion üblichen Lösungsmittel infrage. Hierzu gehören vorzugsweise Ether, wie Diethylether oder Tetrahydrofuran sowie Gemischen mit anderen organischen Solventien, wie z.B. Benzol.

Die Reaktionstemperaturen können beim Verfahren (a) in einem größeren Bereich variiert werden. Im allgemeinen arbeitet man zwischen etwa 20 bis etwa 120 °C, vorzugsweise zwischen etwa 30 bis etwa 80 °C.

Bei der Duchführung des Verfahrens (a) setzt man auf 1 Mol der Verbindung der Formel (II) vorzugsweise einen Ueberschuß von 3 bis 5 Mol der Grignard-Verbindungen der Formel (III) ein. Die Isolierung der Verbindungen der Formel (I) erfolgt in üblicher und bekannter Weise.

Für die erfindungsgemäße Umsetzung nach Verfahren (b) kommen als Verdünnungsmittel vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; aromatische Kohlenwasserstoffe,

wie Benzol, Toluol und Dichlorbenzol; Formamide, wie insbesondere Dimethylformamid; und halogenierte Kohlenwasserstoffe, wie Methylenchlorid, Tetrachlorkohlenstoff oder Chloroform.

Wird das erfindungsgemäße Verfahren (b) in Gegenwart eines Säurebinders vorgenommen, so kann man alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N.N-Dimethycyclohexylamin, Dicyclohexylmethylamin, N,N-Dimethylbenzylamin, weiterhin Pyridin und Diazabicyclooctan. Vorzugsweise verwendet man einen Ueberschuß an Azol.

Die Reaktionstemperaturen können beim Verfahren (b) in einem größeren Bereich variiert werden. Im allgemeinen arbeitet man zwischen etwa 30 bis etwa 200 °C, vorzugsweise bei der Siedetemperatur des Lösungsmittels.

Bei der Durchführung des erfindungsgemäßen Verfahrens (b) setzt man auf 1 Mol der Verbindungen der Formel (IV) vorzugsweise 1 bis 2,5 Mol Azol und 1 bis 2,5 Mol Säurebinder ein. Bei Verwendung eines Alkalisalzes setzt man vorzugsweise auf 1 Mol der Verbindung der Formel (IV) 1 bis 1,5 Mol Alkalisalz ein. Zur Isolierung der Verbindungen der Formel (I) wird das Lösungsmittel abdestilliert, der Rückstand direkt oder nach Aufnahme mit einem organischen Solvens mit Wasser gewaschen, die organische Phase gegebenenfalls über Natriumsulfate getrocknet und im Vakuum vom Lösungsmittel befreit. Der Rückstand wird gegebenenfalls durch Destillation, Umkristallisation oder chromatographisch gereinigt.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Furmarsäure, Weinsäure, Zitonensäure, Salizylsäure, Sorbinsäure, Milchsäure sowie Sulfonsäuren, wie z.B. p-Toluolsulfonsäure und 1,5-Naphthaldisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure. z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere antimykotische Wirkungen auf. Sie besitzen ein sehr breites antimykotisches Wirkungsspektrum, insbesondere gegen Dematophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränk- und der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotiond, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carbosymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quaternäre Ammoniumverbindungen, (g) Netzmittel, z.B. Cetylalkonol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pfanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Aethylalkohol, Isopropylalkohol, Aethylcarbonat, Aethylacetat, Benzylalkohol, Benzylenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstofe sowie geruchs- und geschmackverbessernde Zusätze, z.B. Pfefferminzöl und Eukalyptusöl und Süßmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einingen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicnt erfolgen.

## Beispiel A

### Antimykotische in-vitro-Wirksamkeit

Versuchsbeschreibung :

Die in-vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze : Sabouraud's milieu d'epreuve

b) für Hefen : Fleischextrakt-Traubenzucker-Bouillon.

Die Bebrütungstemperatur betrug 27 °C, die Bebrütungsdauer lag bei 24 bis 96 Stunden.

Bei diesen Tests zeigen die erfindungsgemäßen Verbindungen sehr gute minimale Hemmkonzentrationen und erweisen sich dadurch den bekannten Verbindungen überlegen.

## Beispiel B

### Antimykotische in-vivo-Wirksamkeit (lokal) am Modell der exterimentellen Meerschweinchen-Trichophytie

Versuchsbeschreibung :

Weiße Meerschweinchen der Rasse Pirbright-white wurden auf dem geschorenen, nicht skarifizierten Rücken mit einer Mikro- und Makrokonidien-Suspension von Trichophyton mentagrophytes infiziert. Bei unbehandelten Tieren entwickelt sich innerhalb 12 Tagen p.i. das typische Bild einer Dermatophytose mit Rötung, Schuppung und Haarausfall bis zum totalen Integument-Defekt an der Infektionsstelle. Die infizierten Tiere wurden -beginnend mit dem 3. Tag p.i. — 1 mal täglich mit 1 %-igen Polyethylenglykol-Lösungen der erfindungsgemäßen Präparate lokal behandelt.

Am 14. Tag p.i. zeigten die unbehandelten Kontrolltiere das typische Bild einer Dermatophytose, während z.B. die Herstellungsbeispiele 1 und 3 den Ablauf der Infektion völlig gehemmt hatten.

## Herstellungsbeispiele

### Beispiele 1 und 2

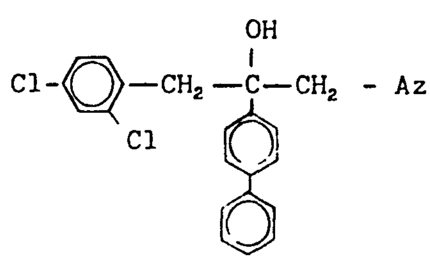

Az = 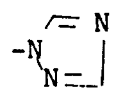 = Beispiel 1

Az = 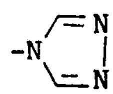 = Beispiel 2

(Verfahren b)

Eine Lösung von 3,5 g (0,065 Mol) Natriummethylat in 18 ml Methylalkohol wird mit 7,6 g (0,11 Mol) 1,2,4-Triazol versetzt ; anschließend wird eine Lösung von 19,5 g (0,05 Mol) 2-(4-Biphenylyl)-3-chlor-1-(2,4-dichlorphenyl)-propan-2-ol in 38 ml Dimethylformamid zugetropft und 90 Mintuten auf 70 °C erhitzt. Die Lösungsmittel werden im Vakuum am Rotationsverdampfer entfernt und der Rückstand mit Wasser verrührt. Die resultierenden Kristalle werden mit Diethylether gewaschen und aus Acetonitril umkristallisiert. Man erhält 2,2 g 2-(4-Biphenylyl)-1-(2,4-dichlorphenyl)-3-(1,3,4-triazol-1-yl)-propan-2-ol vom Schmelzpunkt 260 °C.

Die Acetonitril-Lösung (Mutterlauge) wird eingedampft und die resultierenden Kristalle werden mit Diethylether und Essigester gewaschen. Man erhält 6,5 g 2-(4-Biphenylyl)-1-(2,4-dichlorphenyl)-3-(1,2,4-triazol-1-yl)-propan-2-ol vom Schmelzpunkt 124 °C.

Herstellung des Ausgangsproduktes

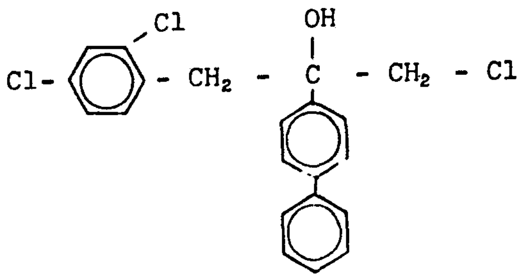

Zu einer Lösung von 0,6 Mol 2,4-Dichlorbenzylmagnesiumchlorid, erhalten aus 15,9 g (0,65 Mol) Magnesium und 117,3 g (0,6 Mol) 2,4-Dichlorbenzylchlorid in 300 ml Ether, werden 69,3 g (0,3 Mol) 4-Phenylphenacylchlorid portionsweise zugegeben. Anschließend wird das Reaktionsgemisch auf wässrige Ammoniumchlorid-Lösung gegossen, die Ether-phase abgetrennt, mit Wasser gewaschen, über Natriumsulfat getrocknet und eingedampft. Das verbleibende Oel wird mit Petrolether extrahiert und die Petroletherlösung eingedampft. Die Kristalle werden abfiltriert und getrocknet. Man erhält 62 g (53 % der Theorie) 2-(4-Biphenylyl)-3-chlor-1-(2,4-dichlorphenyl)-propan-2-ol vom Schmelzpunkt 84 °C.

Beispiele 3 und 4

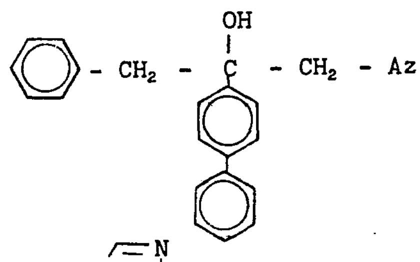

Az = 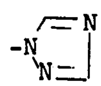 = Beispiel 3

Az = 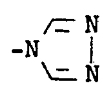 = Beispiel 4

(Verfahren b)

Eine Lösung von 17,9 g (0,33 Mol) Natriummethylat in 90 ml Methylalkohol wird mit 38,3 g (0,56 Mol) 1,2,4-Triazol versetzt ; anschließend wird eine Lösung von 82 g (0,254 Mol) 2-(4-Biphenylyl)-3-chlor-1-phenyl-propan-2-ol in 191 ml Dimethylformamid zugetropft und 90 Minuten auf 60 °C erhitzt. Die Lösungsmittel werden im Vakuum am Rotationsverdampfer entfernt ; der Rückstand wird in Methylenchlorid gelöst und mit Wasser gewaschen. Die Methylenchlorid-Lösung wird über Natriumsulfat getrocknet, filtriert und im Vakuum eingeengt. Das resultierende Oel wird chromatographisch getrennt. Man erhält 13,8 g 2-(4-Biphenylyl)-1-phenyl-3-(1,2,4-triazol-1-yl)-propan-2-ol vom Schmelzpunkt 124 °C und 2,7 g 2-(4-Biphenylyl)-1-phenyl-3-(1,3,4-triazol-1-yl)-propan-2-ol vom Schmelzpunkt 246 °C.

Herstellung des Ausgangsproduktes

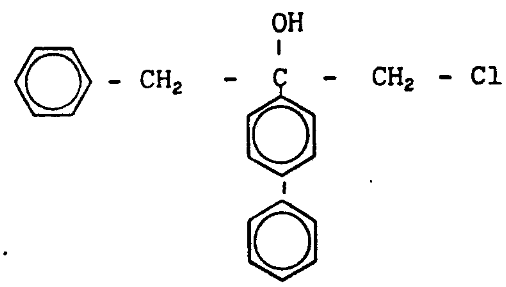

Zu einer Lösung von Benzylmagnesiumchlorid, erhalten aus 24,3 g (1 Mol) Magnesium und 115 ml (1 Mol) Benzylchlorid in 150 ml Diethylether, werden 115,3 g (0,5 Mol) 4-Phenylphenacylchlorid potionsweise zugegeben. Das Reaktionsgemisch wird 90 Minuten rückfließend erwärmt und anschliessend auf wässrige Ammoniumchlorid-Lösung gegossen. Die abgetrennte Etherphase wird mit Wasser gawaschen, über Natriumsulfat getrocknet und eingeengt. Das verbleibende Oel wird durch Verrühren mit Petrolether zur Kristallisation gebracht. Man erhält 50 g (31 % der Theorie) 2-(4-Biphenylyl)-3-chlor-1-phenyl-propan-2-ol vom Schmelzpunkt 96 °C.

In entsprechender weise werden sowohl nach Verfahren (a) als auch nach Verfahren (b) die Verbindungen der nachfolgenden Tabelle 1 erhalten.

Tabelle 1

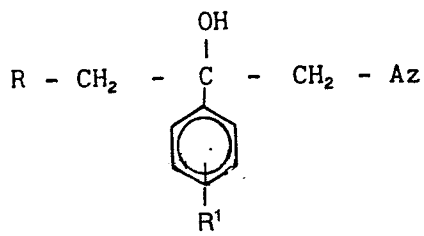

| Bsp. Nr. | R | $R^1$ | Az | Schmelzpunkt (°C) bzw. Brechungsindex |
|---|---|---|---|---|
| 5 | 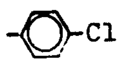 | 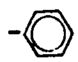 | 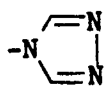 | 250 |
| 6 | 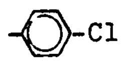 | 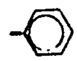 | 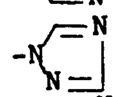 | 104 |
| 7 | 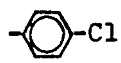 | 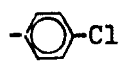 | 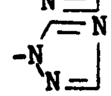 | 144 |
| 8 | 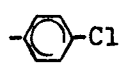 | 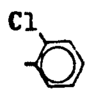 | 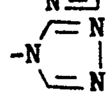 | 175 |
| 9 | 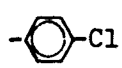 | 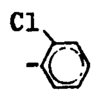 | 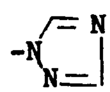 | $n_D^{20}$ : 1,5992 |
| 10 | 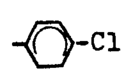 | 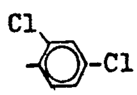 | 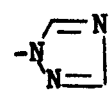 | 160 |
| 11 | 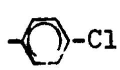 | 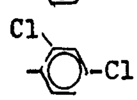 | 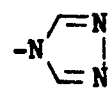 | |
| 12 | 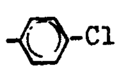 | 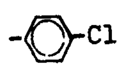 | 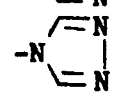 | |

**Ansprüche**

1. Hydroxypropyl-triazole der Formel

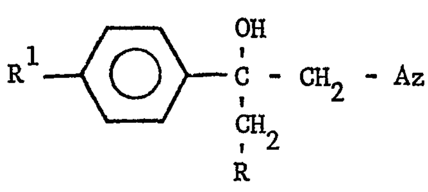 (I)

in welcher

Az für 1,2,4-Triazol-1-yl oder 1,3,4-Triazol-1-yl steht, und

R und $R^1$ für gegebenenfalls ein- oder zweifach durch Chlor substituiertes Phenyl stehen,

und deren physiologisch verträglichen Säureadditions-Salze.

2. Verfahren zur Herstellung von Hydroxypropyl-triazolen der Formel (I) gemäß Anspruch 1, dadurch gekennzeichnet, daß man

a) Triazolylmethyl-phenyl-ketone der Formel

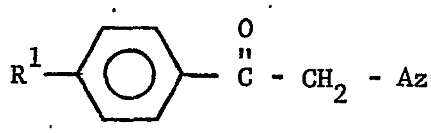

(II)

in welcher

Az und $R^1$ die in Anspruch 1 angegebene Bedeutung haben,

mit einer Grignard-Verbindung der Formel

$$R - CH_2 - Mg - X \qquad (III)$$

in welcher

R die in Anspruch 1 angegebene Bedeutung hat, und

X für Halogen, insbesondere Chlor oder Brom steht,

in Gegenwart eines Verdünnungsmittels umsetzt, oder

b) 1-Halogen-propan-2-ole der Formel

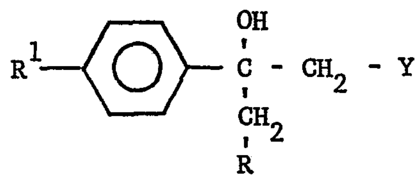

(IV)

in welcher

R und $R^1$ die in Anspruch 1 angegebene Bedeutung haben, und

Y für Halogen, insbesondere Chlor oder Brom steht,

mit Triazol, gegebenenfalls in Gegenwart eines Säurebindemittels und gegebenenfalls in Gegenwart eines Verdünnungsmittels, umsetzt und gegebenenfalls die Verbindungen der Formel (I) durch Umsetzen mit Säuren in die Salze überführt.

3. Arzneimittel, gekennzeichnet durch einen Gehalt an mindestens einem Hydroxypropyltriazol gemäß Anspruch 1.

4. Verfahren zur Herstellung von antimykotischen Mitteln, dadurch gekennzeichnet, daß man Hydroxypropyltriazole gemäß Anspruch 1 mit inerten, nichttoxischen, pharmazeutisch geeigneten Trägerstoffen vermischt.

**Claims**

1. Hydroxypropyl-triazoles of the formula

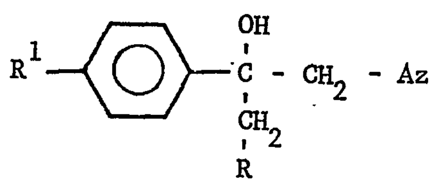

(I)

in which

Az represents 1,2,4-triazol-1-yl or 1,3,4-triazol-1-yl and

R and $R^1$ represent phenyl which is optionally monosubstituted or disubstituted by chlorine, and physiologically acceptable acid addition salts thereof.

2. Process for the preparation of hydroxypropyl-triazoles of the formula (I) according to Claim 1, characterised in that

a) triazolylmethyl phenyl ketones of the formula

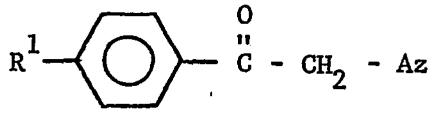

(II)

in which

Az and $R^1$ have the meaning indicated in Claim 1, are reacted with a Grignard compound of the formula

$$R - CH_2 - Mg - X \quad (III)$$

in which

R has the meaning indicated in Claim 1, and

X represents halogen, in particular chlorine or bromine,

in the presence of a diluent, or

b) 1-halogeno-propan-2-ols of the formula

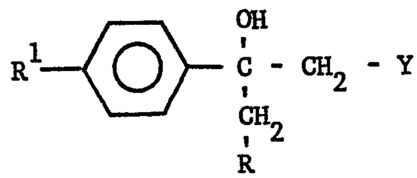

(IV)

in which

R and $R^1$ have the meaning indicated in Claim 1 and

Y represents halogen, in particular chlorine or bromine,

are reacted with triazole, if appropriate in the presence of an acid-binding agent and if appropriate in the presence of a diluent and the compounds of the formula (I) are optionally converted into salts by reaction with acids.

3. Medicaments, characterised in that they contain at least one hydroxypropyltriazole according to Claim 1.

4. Process for the preparation of antimycotic agents, characterised in that hydroxypropyltriazoles according to Claim 1 are mixed with inert, non toxic, pharmaceutically suitable excipients.

**Revendications**

1. Hydroxypropyl-triazoles de formule

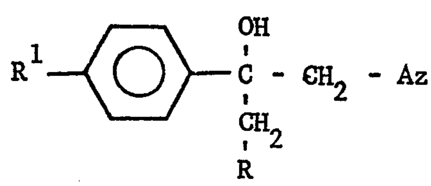

(I)

dans laquelle

Az représente un reste 1,2,4-triazole-1-yle ou 1,3,4-triazole-1-yle et

R et $R^1$ représentent un phényle éventuellement substitué une ou deux fois par le chlore,

et leurs sels d'addition d'acides physiologiquement acceptables.

2. Procédé pour la préparation d'hydroxypropyl-triazoles de formule (I) selon la revendication 1, caractérisé en ce que l'on fait réagir :

a) des triazolylméthylphényl-cétones de formule

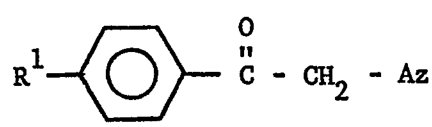

(II)

dans laquelle

Az et $R^1$ ont la signification indiquée à la revendication 1,

avec un composé de Grignard de formule

$$R - CH_2 - Mg - X \quad (III)$$

dans laquelle

R a la signification indiquée à la revendication 1 et

X représente un halogène, en particulier le chlore ou le brome,

en présence d'un agent diluant, ou bien

b) des 1-halogéno-propane-2-ols de formule

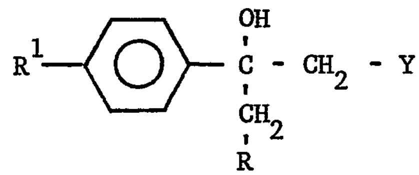

(IV)

dans laquelle

R et $R^1$ ont la signification indiquée à la revendication 1 et

Y représente un halogène, en particulier le chlore ou le brome,

avec le triazole, éventuellement en présence d'un accepteur d'acide et éventuellement en présence d'un agent diluant, et on transforme éventuellement les composés de formule (I) en les sels par réaction avec des acides.

3. Médicaments caractérisés en ce qu'ils contiennent au moins un hydroxypropyltriazole selon la revendication 1.

4. Procédé pour la préparation d'agents antimycotiques, caractérisé en ce que l'on mélange des hydroxypropyl-triazoles selon la revendication 1, avec des supports inertes non toxiques appropriés en pharmacie.